# FASCICULE DE BREVET EUROPEEN

(11) **EP 0 680 748 B1**
(45) Date de publication et mention de la délivrance du brevet: **27.11.1996**
(21) Numéro de dépôt: 95400691.2
(22) Date de dépôt: 28.03.1995
(51) Int. Cl.: A61K 7/48

(54) **Composition cosmétique et/ou dermatologique à gélifiant polymèrique cationique, ses utilisations notamment pour la dépigmentation de la peau**
Kosmetische und/oder dermatologische Zusammensetzung, die ein kationisches polymerisches Geliermittel enthält sowie dessen Verwendung, insbesondere zur Hautdepigmentierung
Cosmetic and/or dermatological composition containing a cationic polymeric gelling agent and its uses especially for skin depigmentation

(30) Priorité: 05.05.1994 FR 9405538
(43) Date de publication de la demande: 08.11.1995
(73) Titulaire: L'OREAL, S.A., 75008 Paris (FR)
(72) Inventeur: Arnaud-Sebillotte, Laurence, F-94000 Creteil (FR); Segot, Evelyne, F-94130 Nogent-sur-Marne (FR)
(74) Mandataire: Lhoste, Catherine

(56) Documents cités:
- EP-A- 0 395 282
- EP-A- 0 494 554
- WO-A-93/07903
- CHEMICAL ABSTRACTS, vol. 118, no. 12, 22 Mars 1993, Columbus, Ohio, US; abstract no. 109406, Y. UCHIYAMA 'Cationic gels for cosmetics'
- POLYMER INTERNATIONAL, vol.30, 1993, GREAT BRITAIN pages 525 - 531 N. WATANABE 'Characteristics of Water-absorbent Polymer Emulsions'

## Description

L'invention se rapporte à une composition cosmétique et/ou dermatologique destinée plus spécialement à la dépigmentation de la peau, aussi bien du visage que du corps humain voire même du cuir chevelu.

L'invention se rapporte aussi à une utilisation de cette composition pour le traitement cosmétique de la peau et en particulier pour dépigmenter la peau, à l'utilisation de cette composition pour la préparation d'une pommade ou d'un onguent destiné au traitement dermatologique de la peau et à un procédé de traitement cosmétique de la peau.

A différentes périodes de leur vie, certaines personnes voient apparaître sur la peau et plus spécialement sur les mains des taches plus foncées et/ou plus colorées, conférant à la peau une hétérogénéité. En général, ces taches sont dues à une production importante de mélanine dans l'épiderme et/ou le derme de la peau.

Ces taches peuvent être liées à plusieurs phénomènes et plus spécialement au vieillissement. Dans certains cas, ces taches peuvent devenir cancéreuses. Aussi, on cherche de plus en plus à diminuer, voire éliminer, ces taches.

Les compositions classiquement utilisées dans les domaines cosmétique et/ou dermatologique sont des émulsions eau-dans-huile (E/H), des émulsions huile-dans-eau (H/E), ou des gels aqueux, dans lesquels il est souvent difficile, voire même impossible d'incorporer des actifs dépigmentants comme l'acide kojique, l'acide caféique, l'acide salicylique et ses dérivés mais aussi d'autres actifs comme les filtres solaires, les agents amincissants, les agents anti-radicaux libres, les vitamines, les répulsifs pour insectes, utilisés dans différents types de traitement comme la lutte contre le vieillissement, l'acné, les surcharges pondérales ou pour favoriser la circulation sanguine, etc.

En général, ces actifs ont tendance à recristalliser ou à se dégrader. Il s'ensuit une perte d'efficacité plus ou moins importante de ces compositions, selon le degré de recristallisation et/ou de dégradation, ce qui va à l'encontre de l'objectif recherché. En outre, cette recristallisation ou dégradation peut modifier la stabilité globale de ces compositions ainsi que leur aspect, ce qui peut détourner l'utilisateur de ces compositions à traitement spécifique. Par ailleurs leur dissolution dans une composition quelconque nécessite souvent de chauffer cette dernière, ce qui est relativement gênant pour des actifs sensibles à la chaleur comme les vitamines, certains extraits de plante ou certaines protéines.

Pour solubiliser certains de ces actifs, il est connu d'utiliser des émulsions E/H ou H/E dans lesquelles la phase aqueuse présente un pH acide. Pour que ces émulsions soient stables (non séparation des phases aqueuse et huileuse), il est nécessaire d'utiliser des émulsionnants (ou tensioactifs). Malheureusement, ces tensioactifs sont souvent irritants pour la peau. En outre ces émulsions manquent souvent de fraîcheur à l'application, ce qui peut gêner leurs utilisations pendant les périodes chaudes de l'année et/ou dans les pays chauds. Un gel aqueux est beaucoup plus apprécié dans ces conditions d'utilisation. Mais sa trop grande quantité d'eau ne permet pas d'y introduire ces actifs.

En outre, le document EP-A-671157 opposable au titre de l'article 54 (3) CBE décrit des compositions épaissies par un polymère cationique, comprenant moins de 20 % de solvant.

Il subsiste donc le besoin d'une composition stable ayant l'aspect d'un gel, utilisable notamment dans les domaines cosmétique et/ou dermatologique permettant une solubilisation suffisante des actifs généralement utilisés dans ces domaines en vue d'une efficacité maximum.

De façon surprenante, la demanderesse a trouvé qu'il était possible de solubiliser une grande quantité d'actif dans une composition cosmétique et/ou dermatologique ayant, entre autre, toutes les propriétés d'un gel.

Ainsi, l'invention a pour objet une composition telle que définie dans la revendication 1.

La composition de l'invention a une certaine consistance et/ou tenue ; elle n'est pas filante, c'est-à-dire qu'elle ne forme pas de fil lorsqu'on la prend au doigt. Elle se présente plus spécialement sous forme d'un gel.

Cette composition, contrairement aux émulsions, ne contient peu ou pas de tensioactifs. Par ailleurs, contrairement aux processus d'obtention classiques des compositions gélifiées ou émulsionnées, il n'est pas nécessaire de chauffer pour former la composition. Ceci facilite donc sa fabrication et permet d'utiliser des actifs sensibles à la chaleur comme les vitamines ou les extraits de végétaux. Il s'ensuit donc une efficacité améliorée par rapport aux compositions obtenues par chauffage.

Cette composition est particulièrement bien adaptée à la solubilisation d'actifs utilisés dans les domaines cosmétique et dermatologique. Aussi, la composition de l'invention contient, en outre, au moins un actif choisi notamment parmi les actifs dépigmentants, kératolytiques, amincissants, de traitement de l'acné, anti-inflammatoires.

Comme actifs solubilisables dans la composition de l'invention, on peut citer l'acide ascorbique, l'acide kojique, l'acide citrique, l'acide caféique, l'acide salicylique et ses dérivés (par exemple acide n-octanoyl-5 ou décanoyl-5-salicylique), les α-hydroxyacides tels que l'acide lactique, l'acide méthyllactique, l'acide glucuronique, l'acide glycolique, l'acide pyruvique, l'acide 2-hydroxybutanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxynonanoïque, l'acide 2-hydroxydécanoïque, l'acide 2-hydroxyundécanoïque, l'acide 2-hydroxydodécanoïque, l'acide 2-hydroxytétradécanoïque, l'acide 2-hydroxyhexadécanoïque, l'acide 2-hydroxyoctadécanoïque, l'acide 2-hydroxytétraécosanoïque, l'acide 2-hydroxyeïcosanoïque, l'acide mandélique, l'acide benzoïque, l'acide phényllactique, l'acide gluconique, l'acide galacturonique, l'acide aleuritique, l'acide ribonique, l'acide tartronique, l'acide tartrique, l'acide malique, l'acide fumarique, l'acide rétinoïque et ses dérivés, l'acide benzène 1,4-di(3-méthylidène 10-camphosulfonique). On peut aussi, utiliser tous les composés naturels ou synthétiques contenant de tels acides, comme les extraits végétaux et plus spécialement les extraits de fruits. On peut aussi solubiliser les dérivés xanthiques (caféine, théophylline), l'acide β-glycyrrhétinique, l'acide asiatique, l'octopirox ou encore le rétinol et ses esters.

Il est tout à fait surprenant d'obtenir une composition parfaitement stable, avec le polymére de l'invention, connu pour épaissir un milieu hydrophile contenant une grande quantité d'eau (voir notamment le document EP-A-395282). En effet, rien ne permettait de prévoir que cet épaississant puisse gélifier et stabiliser efficacement une composition aussi pauvre en eau, et en particulier contenant au plus 30 % d'eau. Par ailleurs, ce copolymère favorise la solubilisation des actifs ci-dessus.

Certes, il est connu de stabiliser des compositions cosmétiques avec des gélifiants comme les polymères naturels du type dérivés de cellulose (hydroxypropyl cellulose, hydroxypropyl méthylcellulose, hydroxyéthylcellulose alkylé), les dérivés de polyolosides (hydroxypropylguar), ou comme les polymères de synthèse tels que le copolymère métho-chlorure de vinylimidazolinium/vinyl pyrrolidone et le copolymère ammonium acrylate/acrylonitrogène. Mais ces gélifiants, qui ont été testés par la demanderesse, ne sont nullement appropriés aux compositions acides et riches en solvant de l'invention.

Par composition stable, il faut comprendre une composition restant homogène et dans laquelle le ou les actifs ne recristallisent et/ou ne se dégradent pas, notamment pendant au moins 2 mois à 45 ° C.

De façon avantageuse, le pH de la phase aqueuse est choisi inférieur ou égal à 4, et en pratique choisi dans la gamme allant de 1 à 4. Il est toutefois possible d'avoir un pH allant jusqu'à 5.

Le ou les gélifiants de l'invention sont notamment des copolymères ou des homopolyméres cationiques réticulés, substantiellement solubles dans le milieu hydrophile et notamment dans l'eau et constitués de motifs résultant de la reaction entre (i) un monomère cationique à insaturation éthylénique ou un mélange cationique de monomères à insaturation éthylénique et (ii) un agent de réticulation à polyinsaturation éthylénique, cet agent de réticulation étant présent dans le polymère à une concentration allant de 5 ppm à 45ppm, de préférence de 10 à 40 ppm et mieux encore de 10 à 20 ppm.

Les polymères ci-dessus peuvent ainsi être obtenus classiquement selon la technique dite de polymérisation en émulsion à partir des différents monomères rentrant dans leur constitution.

Les monomères à polyinsaturation éthylénique utilisés comme agent de réticulation pour la préparation des polymères conformes à l'invention sont de préférence choisis dans le groupe constitué par le méthylène bis-acrylamide, l'éthylène glycol di-(méth)acrylate, le di-(méth)acrylamide, le cyanométhylacrylate, le vinyloxyéthyl(méth)acrylate, ou leurs seuls métalliques. Encore plus préférentiellement, on fait appel au méthylène bis-acrylamide.

Le mélange cationique de monomères peut contenir, en plus des monomères cationiques, des monomères non-ioniques.

Les monomères cationiques utilisables dans l'invention sont notamment les di-alkylaminoalkyl-acrylates et méthacrylates, et plus spécialement les di-alkylaminoéthyl (meth)acrylates, et leurs sels quaternaires ou acides ; les dialkylaminoalkyl-acrylamides ou méthacrylamides et leurs sels quaternaires ou acides comme le chlorure de méthacrylamidopropyl triméthyl ammonium. Les groupes alkyle ont par exemple de 1 à 4 atomes de carbone.

Les monomères non-ioniques utilisables en association avec les monomères cationiques sont par exemple le méthacrylamide ou l'acrylamide.

Les polymères cationiques utilisables dans l'invention sont en particulier ceux décrit dans le document EP-A-395 282.

De préférence, le polymère cationique est constitué d'au moins un homopolymère de chlorure de diméthylaminoéthyl méthacrylate de méthyle ou d'un polyméthacrylate de diméthylaminoéthyl quaternisé.

Plus particulièrement, on utilise les polymères commercialisés sous les noms de Salcare SC 92 ^{R} ou Salcare SC 95 ^{R} par la société Allied Colloids Limited. On peut aussi utiliser ceux vendus sous le nom de Pas 5194 par la société Hoechst.

Selon l'invention, il est possible d'associer à ce polymère d'autres gélifiants hydrosolubles tels que les gommes de cellulose, les polyuréthannes, les dérivés acryliques comme par exemple le polyméthacrylate de glycéryle à 2 % dispersé dans un mélange eau/glycérine vendu sous le nom Norgel ^{R} ou Lubrajel ^{R} par la société Guardian.

La quantité de polymère cationique, selon l'invention, est choisie, en matière active, par exemple de 0,1 % à 10 % du poids total de la composition et de préférence de 0,5 % à 6 % et mieux encore de 1 % à 4 % ; ceci correspond à environ 0,1 % à 10 % du poids total de la composition, pour la formulation commerciale du copolymère, et de préférence de 1 % à 12 % et mieux encore de 2 % à 8 %.

La quantité, en matière active, de gélifiants additionnels est choisie par exemple de 0 % à 10 % du poids total de la composition, de préférence de 0,1 % à 6 % et mieux de 0,5 % à 3 %.

Les solvants utilisables dans l'invention sont des solvants hydrophiles associés éventuellement à des solvants lipophiles ou à la fois hydrophiles et lipophiles. En particulier, ces solvants sont des solvants cosmétiquement et/ou dermatologiquement acceptables (tolérance, toxicologie et toucher acceptables).

Les solvants hydrophiles peuvent représenter de 20 % à 70 % et mieux de 50 % à 70 % du poids total de la composition. Ces solvants hydrophiles sont par exemple des mono-alcools inférieurs linéaires ou ramifiés ayant de 1 à 8 atomes de carbone comme l'éthanol, le propanol, le butanol, l'isopropanol, l'isobutanol ; des polyéthyléne glycols ayant de 6 oxydes d'éthylène à 80 oxydes d'éthylène ; des polyols tels que le propylène glycol, l'isoprène glycol, le butylène glycol, le glycérol, le sorbitol ; les mono- ou di-alkyle d'isosorbide dont les groupements alkyle ont de 1 à 5 atomes de carbone comme le diméthyl isosorbide ; les éthers de glycol comme le diéthylène glycol mono-méthyl ou mono-éthyl éther et les éthers de propylène glycol comme le dipropylène glycol méthyl éther.

De préférence, la composition de l'invention contient au moins 10 % en poids, par rapport au poids total de la composition, d'alcools inférieurs.

Comme solvants à la fois lipophiles et hydrophiles, on peut citer des polyols tels que l'isoprène glycol ; des dérivés de polypropylène glycol (PPG) tels que les esters de polypropylène glycol et d'acide gras, de PPG et d'alcool gras comme le PPG-23 oléyl éther et le PPG-36 oléate. Ces solvants sont utilisés en combinaison avec les solvants hydrophiles avec une concentration allant de 0 % à 50 % du poids total de la composition et de préférence de 0 % à 30 % et mieux de 0 à 10 %.

On peut éventuellement utiliser des solvants lipophiles associés aux solvants hydrophiles et/ou à la fois hydrophiles et lipophiles. Ces solvants lipophiles représentent de 0 % à 50 % du poids total de la composition et de préférence de 0 % à 30 % et mieux de 0 à 10 %. Ces solvants sont en particulier des esters gras tels que l'adipate de diisopropyle, l'adipate de dioctyle, le malate de dioctyle, l'isostéarate de glycéryle, l'acétostéarate d'octyle, l'octanoate de cétyle, le sébacate de diisopropyle ; des alcools de Guerbet (ou 2-alkyl-1-alcanols) de structure générale R₁-CHR₂-CH₂OH avec R₁ et R₂ identiques ou différents représentant un atome d'hydrogène ou un radical alkyle ayant de 1 à 10 atomes de carbone comme l'octyldodécanol, l'hexyldécanol, le cyclododécanol.

Afin que la composition de l'invention soit plus agréable à utiliser (plus douce à l'application, plus nourrissante, plus émolliente), il est possible d'ajouter une ou plusieurs huiles de préférence solubles dans le milieu hydrophile, c'est-à-dire dans l'eau ou le solvant.

Les huiles solubles dans l'eau ou dans le milieu hydroalcoolique représentent de 0 % à 50 % du poids total de la composition, de préférence de 0 % à 30% et mieux de 1 % à 15 %.

Les huiles partiellement solubles dans le solvant, utilisables dans l'invention, sont par exemple les cyclométhicones (cyclohexa-, cyclopenta- ou cyclotétra-diméthylsiloxane), les polydiméthyl siloxanes volatils, linéaires (à 2, 3, ou 4 fonctions siloxane) ou encore les phényldiméthyl siloxanes.

Les huiles solubles dans l'eau, utilisables dans l'invention, sont par exemple les silicones hydrosolubles telles que les :
- polydiméthyl siloxanes oxyéthylénés à motifs amide comme par exemple ceux vendus sous le nom Silwax AX^{R} ou Silwax DCA-100^{R} par la société Siltech ;
- polydiméthyl siloxanes oxyéthylénés à groupements stéarate comme par exemple ceux vendus sous le nom Silwax WD-IS ^{R} par la société Siltech ;
- polydiméthyl siloxanes oxyéthylénés comme par exemple ceux vendus sous le nom Belsil DMC 6038 ^{R} par la société Wacker ;
- stéaroxy polydiméthyl siloxanes comme par exemple ceux vendus sous le nom Belsil SDM 6022 ^{R} par la société Wacker.

On peut aussi utiliser des huiles insolubles dans le milieu hydroalcoolique. Ces huiles représentent de 0 % à 20 % et de préférence de 1 % à 15 %. Ces huiles insolubles sont par exemples :
- les huiles minérales telles que l'huile de paraffine et de vaseline,
- les huiles d'origine animale telles que le perhydrosqualène,
- les huiles d'origine végétale telles que l'huile d'amande douce, l'huile d'avocat, l'huile de ricin, l'huile d'olive, l'huile de jojoba, l'huile de sésame, l'huile d'arachide, l'huile de macadamia, l'huile de pépins de raisin, l'huile de colza, l'huile de coprah, l'huile de noisette, le beurre de karité et sa fraction liquide, l'huile de palme, l'huile de noyau d'abricot, l'huile de calophyllum, l'huile de son de riz, l'huile de germe de maïs, l'huile de germe de blé, l'huile de soja, l'huile de tournesol, l'huile d'onagre, l'huile de carthame, l'huile de passiflore et l'huile de seigle,
- les huiles synthétiques telles que l'huile de purcellin, les esters gras dont le myristate de butyle, le myristate d'isopropyle, le stéarate d'héxadécyle, le stéarate d'isopropyle, le stéarate d'octyle, le stéarate d'isocétyle, l'oléate de décyle, le laurate d'héxyle, le dicaprylate de propylène glycol et les esters dérivés d'acide lanolique tels que le lanolate d'isopropyle, le lanolate d'isocétyle et les isoparaffines, les huiles fluorées et perfluorées, et enfin les huiles siliconées, volatiles ou non.

Les huiles utilisées peuvent également contenir un ou plusieurs actifs cosmétiques et/ou thérapeutiques lipophiles, notamment ceux utilisés de manière habituelle dans la fabrication et l'obtention des compositions cosmétiques et/ou pharmaceutiques. Ces actifs peuvent être, par exemple, des agents anti-radicaux libres ; des céramides ; des filtres solaires (notamment ultraviolets) tels que le paraméthoxycinnamate de 2-éthylhexyle et en particulier celui commercialisé sous la dénomination "Parsol MCX ^{R} " par la Société GIVAUDAN ou la 2-hydroxy-4-méthoxybenzophénone et en particulier celle vendue sous la dénomination commerciale "Uvinul M40 ^{R} " par la Société BASF ; des répulsifs pour insectes tels que le cétyl-3 éthylaminopropionate de n-butyle ou le diéthylamide-N,N-caprylique ; des agents amincissants tels que le nicotinate de D-L α-tocophérol, l'extrait huileux de ginseng (Panax Ginseng), l'extrait huileux de lierre grimpant (Hedera Helix), l'extrait huileux de fleurs sèches d'arnica (Arnica montana L) et l'extrait huileux d'algues (Fucus Vesiculosus). Il va de soi que la liste ci-dessus d'actifs lipophiles susceptibles d'être introduits dans la composition de l'invention n'est nullement exhaustive.

Les huiles peuvent également contenir, selon l'application envisagée, un ou plusieurs additifs de formulation lipophiles tels que des agents conservateurs, antioxydants ou émollients, des huiles parfumées voire même des parfums.

L'invention a encore pour objet l'utilisation cosmétique de la composition ci-dessus pour traiter la peau du visage et/ou du corps humain et notamment pour dépigmenter la peau du visage et/ou du corps humain et plus spécialement les mains.

L'invention se rapporte aussi à un procédé de traitement cosmétique, par voie topique, de la peau, qui consiste à appliquer sur la peau, du visage et/ou du corps humain une composition telle que définie précédemment. Le type de traitement est fonction du ou des actifs solubilisés dans la composition.

Plus spécialement, l'invention se rapporte à un procédé cosmétique pour dépigmenter la peau du visage et/ou du corps humain, consistant à appliquer sur la peau une composition telle définie ci-dessus contenant au moins un agent dépigmentant solubilisé.

L'invention a encore pour objet une utilisation de la composition ci-dessus pour préparer une pommade ou un onguent destiné à traiter thérapeutiquement le visage et/ou le corps humain, y compris les mains.

L'invention a encore pour objet une utilisation d'un polymère tel que défini précédemment, pour solubiliser un actif dans une composition homogène macroscopiquement, contenant un milieu hydrophile acide, contenant une quantité de solvant organique représentant de 20 à 90 % du poids total de la composition et renfermant une quantité d'eau représentant au plus 45% du poids total de la composition et/ou pour gélifier ladite composition.

La description qui suit est donnée à titre illustratif. Les concentrations des différents constituants entrant dans les compositions exemplifiées ci-après, sont données en pourcentage pondéral. Ces différentes compositions ont été obtenues selon le mode opératoire suivant.

On solubilise le ou les actifs dans le solvant ou le mélange de solvants choisi (phase A), puis on gélifie avec le copolymère (phase C) par simple homogénéisation à température ambiante au moyen d'une turbine à hélice. Les huiles émollientes (phase B) sont ensuite incorporées, toujours à température ambiante de la même façon que pour le copolymère. Cette mise en oeuvre est vraiment simple à réaliser, ce qui est, pour une fabrication en série, un gros avantage, diminuant le prix de revient des produits finaux.

Les compositions obtenues sont soit fluides et non filantes, soit avec un seuil d'écoulement tel que le produit ne s'écoule pas sous son propre poids. Elles sont onctueuses et peuvent avoir un aspect de gel transparent ou translucide ou l'aspect d'une crème opaque. Ces compositions sont toutes des compositions dépigmentantes, mais il va de soi que l'invention est d'application beaucoup plus générale comme indiqué précédemment.

### EXEMPLE 1 : GEL DEPIGMENTANT

| ***Phase A*** | |
|---|---|
| Eau déminéralisée | qsp 100 % |
| Alcool éthylique à 96 ° dans de l'eau | 30 % |
| Polyéthylène glycol 8 fois oxyéthyléné | 30 % |
| Acide kojique | 1 % |
| Acide caféique | 1,2 % |
| Acide n-octanoyl-5-salicylique | 1,5 % |

| ***Phase B*** | |
|---|---|
| Polydiméthyl siloxane oxyéthyléné (Belsil DMC 6038) | 2 % |

| ***Phase C*** | |
|---|---|
| Salcare SC 95 ^{R} (*) | 5 % |

Le produit obtenu a l'aspect d'un gel translucide, lisse, brillant et stable. Son pH est de 3,1. Il peut être appliqué chaque jour sur les taches du visage et du cou en vue de les diminuer.

(*) le Salcare SC 95 ^{R} est un chlorure d'ammonium quaternaire homopolymérique (polyquaternium-37 selon la nomenclature CTFA 5^{ieme} édition) dans un mélange d'huile minérale, de PPG-1 Trideceth-6 (CTFA 5^{ieme} édition).

### EXEMPLE 2 : GEL DEPIGMENTANT

| ***Phase A*** | |
|---|---|
| Eau déminéralisée | qsp 100 % |
| Alcool éthylique à 96 ° dans de l'eau | 30 % |
| Polyéthyléne glycol 8 fois oxyéthyléné | 30 % |
| Acide kojique | 1 % |
| Acide caféique | 0,2 % |
| Acide salicylique | 1 % |

| ***Phase B*** | |
|---|---|
| Cyclohexadiméthylsiloxane | 5 % |

| ***Phase C*** | |
|---|---|
| Salcare SC 95 ^{R} | 4 % |

| ***Phase D*** | |
|---|---|
| Polyméthacrylate de glycéryle à 2 % dans un mélange eau/glycérine (Norgel ^{R}) | 20 % |

La phase D est introduite dans le mélange A+B+C à température ambiante par simple homogénéisation.

Le produit obtenu a l'aspect d'un gel translucide, lisse, brillant et stable. Son pH est de 3,5. Il peut être appliqué une ou deux fois par jour sur les taches du visage et/ou du cou en vue de les diminuer.

### EXEMPLE 3 : GEL ANTI-TACHES POUR LES MAINS

| ***Phase A*** | |
|---|---|
| Eau déminéralisée | qsp 100 % |
| Octyldodécanol | 30 % |
| Polyéthyléne glycol 8 fois oxyéthyléné | 30 % |
| Acide kojique | 1 % |
| Acide caféique | 1,2 % |
| Acide n-octanoyl-5-salicylique | 0,5 % |

| ***Phase B*** | |
|---|---|
| Huile de macadamia | 2 % |

| ***Phase C*** | |
|---|---|
| Salcare SC 92 ^{R} (*)% | 5 % |

Le produit obtenu a l'aspect d'une crème assez fluide opaque, lisse, brillante et stable. Son pH est de 3,8. Il peut être appliqué chaque jour sur les taches des mains.

(*) le Salcare SC 92 ^{R} est un chlorure d'ammonium quaternaire homopolymérique (polyquaternium-32 selon la nomenclature CTFA 5^{ieme} édition) dans de l'huile minérale.

### EXEMPLE 4 : GEL ANTI-TACHES POUR LES MAINS

| ***Phase A*** | |
|---|---|
| Eau déminéralisée | qsp 100 % |
| Alcool éthylique à 96 °dans l'eau | 30 % |
| Polyéthylène glycol 8 fois oxyéthyléné | 30 % |
| Acide caféique | 1 % |

| ***Phase B*** | |
|---|---|
| Huile d'amande douce | 2 % |

| ***Phase C*** | |
|---|---|
| Salcare SC 95 ^{R} | 8 % |

Le produit obtenu a l'aspect d'un gel/crème opaque, lisse, brillant et stable. Son pH est de 4. Il peut être appliqué chaque jour sur les taches du cou ou du visage.

### EXEMPLE 5 : GEL ANTI-TACHES POUR LES MAINS

| ***Phase A*** | |
|---|---|
| Eau déminéralisée | qsp 100 % |
| Ethanol | 30 % |
| Propylène glycol | 30 % |
| Acide kojique | 2 % |
| Acide salicylique | 1 % |

| ***Phase C*** | |
|---|---|
| Salcare SC 95 ^{R} | 2,5 % |

Le produit obtenu a l'aspect d'un gel translucide, lisse, brillant et stable. Il peut être appliqué chaque jour sur les taches des mains.

## Revendications

1. Composition contenant un milieu hydrophile acide et au moins un agent gélifiant formé d'un polymère cationique réticulé, caractérisée en ce que le milieu hydrophile est un milieu contenant une quantité de solvant organique représentant de 20 à 90 % du poids total de la composition, renfermant une quantité d'eau représentant au plus 45 % du poids total de la composition, le gélifiant conférant à la composition un aspect macroscopiquement homogène de gel et une stabilité.

2. Composition selon la revendication 1, caractérisée en ce que le pH est au plus égal à 4.

3. Composition selon la revendication 1 ou 2, caractérisée en ce que la quantité d'eau représente au plus 30% du poids total de la composition.

4. Composition selon l'une quelconque des revendications précédentes, caractérisée en ce que le polymère est un homopolymère formé par réticulation d'au moins un monomère cationique à insaturation éthylénique ou un de ses sels avec un agent de réticulation à polyinsaturation éthylénique.

5. Composition selon l'une quelconque des revendications précédentes, caractérisée en ce que le polymère est un polymère de dialkylaminoalkyl (méth)acrylate ou d'un de ses sels.

6. Composition selon l'une quelconque des revendications précédentes, caractérisée en ce que le polymère est un polymère de diméthylaminoéthyl méthacrylate éventuellement quaternisé.

7. Composition selon l'une quelconque des revendications précédentes, caractérisée en ce que le polymère est présent (en matière active) à raison de 0,1% à 10% du poids total de la composition.

8. Composition selon l'une quelconque des revendications précédentes, caractérisée en ce que le polymère est présent (en matière active) à raison de 0,5% à 6% du poids total de la composition.

9. Composition selon l'une quelconque des revendications précédentes, caractérisée en ce que le milieu hydrophile contient au moins un solvant organique choisi parmi les alcools mono- ou polyfonctionnels les polyéthylène glycols éventuellement oxyéthylénés, les esters de propylène glycol, le sorbitol et ses dérivés, les di- alkyles d'isosorbide, les éthers de glycol et les éthers de propylène glycol.

10. Composition selon l'une quelconque des revendications précédentes, caractérisée en ce que le solvant représente de 20 % à 70 % du poids total de la composition.

11. Composition selon l'une quelconque des revendications précédentes, caractérisée en ce qu'il contient au moins une huile soluble ou non dans le milieu hydrophile.

12. Composition selon l'une quelconque des revendications précédentes, caractérisée en ce que l'huile représente de 0 % à 50% du poids total de la composition.

13. Composition selon l'une, quelconque des revendications précédentes, caractérisée en ce que l'huile représente de 1% à 15% du poids total de la composition.

14. Composition selon l'une quelconque des revendications précédentes, caractérisée en ce qu'elle contient, en outre, au moins un actif dépigmentant apte à dépigmenter la peau d'un être humain.

15. Composition selon l'une quelconque des revendications précédentes, caractérisée en ce qu'elle contient au moins un actif choisi parmi l'acide ascorbique, l'acide kojique, l'acide citrique, l'acide caféique, l'acide salicylique et ses dérivés, l'acide lactique l'acide méthyllactique l'acide glucuronique, l'acide glycolique, l'acide pyruvique, l'acide 2-hydroxybutanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxynonanoïque, l'acide 2-hydroxydécanoïque, l'acide 2-hydroxyundécanoïque, l'acide 2-hydroxydodécanoïque, l'acide 2-hydroxytétradécanoïque, l'acide 2-hydroxyhexadécanoïque, l'acide 2-hydroxyoctadécanoïque, l'acide 2-hydroxytétraécosanoïque, l'acide 2-hydroxyeïcosanoïque, l'acide mandélique, l'acide benzoïque, l'acide phényllactique, l'acide gluconique, l'acide galacturonique, l'acide aleuritique, l'acide ribonique, l'acide tartronique, l'acide tartrique, l'acide malique, l'acide fumarique, l'acide rétinoïque et ses dérivés, l'acide benzène 1,4-di(3-méthylidène 10-camphosulfonique).

16. Composition selon l'une quelconque des revendications précédentes, caractérisée en ce qu'elle contient au moins un actif choisi parmi l'acide kojique, l'acide caféique, l'acide salicylique et ses dérivés, et leurs mélanges.

17. Composition selon l'une quelconque des revendications précédentes, caractérisée en ce qu'elle contient, encore, au moins un additif choisi parmi les céramides, les agents conservateurs, les agents antioxydants, les agents émollients, les parfums.

18. Composition selon l'une quelconque des revendications précédentes, caractérisée en ce qu'elle consiste en une composition cosmétique et/ou dermatologique.

19. Utilisation cosmétique de la composition selon l'une quelconque des revendications 1 à 18, pour traiter la peau du visage et/ou du corps humain.

20. Utilisation cosmétique de la composition selon l'une quelconque des revendications 1 à 18, pour dépigmenter la peau du visage et/ou du corps humain.

21. Procédé de traitement cosmétique de la peau, caractérisé en ce qu'il consiste à appliquer sur la peau une composition selon l'une quelconque des revendications 1 à 18.

22. Procédé cosmétique pour dépigmenter la peau du visage et/ou du corps humain, caractérisé en ce qu'il consiste à appliquer sur la peau une composition selon l'une quelconque des revendications 1 à 18.

23. Utilisation de la composition selon l'une quelconque des revendications 1 à 18, pour préparer une pommade ou un onguent destiné à traiter thérapeutiquement le visage et/ou le corps humain.

24. Utilisation d'un polymère cationique réticulé, pour solubiliser un actif dans une composition contenant un milieu hydrophile acide, contenant une quantité de solvant organique représentant de 20 à 90 % du poids total de la composition et renfermant une quantité d'eau représentant au plus 45% du poids total de la composition, et/ou pour gélifier ladite composition.

## Claims

1. Composition containing an acidic hydrophilic medium and at least one gelling agent formed of a crosslinked cationic polymer, characterized in that the hydrophilic medium is a medium containing an amount of organic solvent representing from 20 to 90% of the total weight of the composition, including an amount of water representing not more than 45% of the total weight of the composition, the gelling agent imparting a macroscopically homogeneous gel appearance and stability to the composition.

2. Composition according to Claim 1, characterized in that the pH is at most equal to 4.

3. Composition according to Claim 1 or 2, characterized in that the amount of water represents not more than 30% of the total weight of the composition.

4. Composition according to any one of the preceding claims, characterized in that the polymer is a homopolymer formed by crosslinking of at least one cationic monomer containing ethylenic unsaturation or one of its salts with a crosslinking agent containing ethylenic polyunsaturation.

5. Composition according to any one of the preceding claims, characterized in that the polymer is a polymer of dialkylaminoalkyl (meth)acrylate or one of its salts.

6. Composition according to any one of the preceding claims, characterized in that the polymer is an optionally quaternized polymer of dimethylaminoethyl methacrylate.

7. Composition according to any one of the preceding claims, characterized in that the polymer is present (as active material) in a proportion of from 0.1% to 10% of the total weight of the composition.

8. Composition according to any one of the preceding claims, characterized in that the polymer is present (as active material) in a proportion of 0.5% to 6% of the total weight of the composition.

9. Composition according to any one of the preceding claims, characterized in that the hydrophilic medium contains at least one organic solvent chosen from mono-or polyfunctional alcohols, polyethylene glycols which may be oxyethylenated, propylene glycols esters, sorbitol and its derivatives, dialkyl isosorbides, glycol ethers and propylene glycol ethers.

10. Composition according to any one of the preceding claims, characterized in that the solvent represents from 20% to 70% of the total weight of the composition.

11. Composition according to any one of the preceding claims, characterized in that it contains at least one oil which may or may not be soluble in the hydrophilic medium.

12. Composition according to any one of the preceding claims, characterized in that the oil represents from 0% to 50% of the total weight of the composition.

13. Composition according to any one of the preceding claims, characterized in that the oil represents from 1% to 15% of the total weight of the composition.

14. Composition according to any one of the preceding claims, characterized in that it also contains at least one depigmenting active agent capable of depigmenting human skin.

15. Composition according to any one of the preceding claims, characterized in that it contains at least one active agent chosen from ascorbic acid, kojic acid, citric acid, caffeic acid, salicylic acid and its derivatives, glucuronic acid, glycolic acid, pyruvic acid, 2-hydroxybutanoic acid, 2-hydroxypentanoic acid, 2-hydroxyhexanoic acid, 2-hydroxyheptanoic acid, 2-hydroxyoctanoic acid, 2-hydroxynonanoic acid, 2-hydroxydecanoic acid, 2-hydroxyundecanoic acid, 2-hydroxydodecanoic acid, 2-hydroxytetradecanoic acid, 2-hydroxyhexadecanoic acid, 2-hydroxyoctadecanoic acid, 2-hydroxytetraeicosanoic acid, 2-hydroxyeicosanoic acid, mandelic acid, benzoic acid, phenyllactic acid, gluconic acid, galacturonic acid, aleuritic acid, ribonic acid, tartronic acid, tartaric acid, malic acid, fumaric acid, retinoic acid and its derivatives, and benzene-1,4-di(3-methylidene-10 camphorsulphonic acid).

16. Composition according to any one of the preceding claims, characterized in that it contains at least one active agent chosen from kojic acid, caffeic acid, salicylic acid and its derivatives, and mixtures thereof.

17. Composition according to any one of the preceding claims, characterized in that it further contains at least one additive chosen from ceramides, preserving agents, antioxidants, emollients and fragrances.

18. Composition according to any one of the preceding claims, characterized in that it consists of a cosmetic and/or dermatological composition.

19. Cosmetic use of the composition according to any one of Claims 1 to 18 for treating human facial and/or body skin.

20. Cosmetic use of the composition according to any one of Claims 1 to 18 for depigmenting human facial and/or body skin.

21. Cosmetic skin-treatment process, characterized in that it consists in applying a composition according to any one of Claims 1 to 18 to the skin.

22. Cosmetic process for depigmenting human facial and/or body skin, characterized in that it consists in applying a composition according to any one of Claims 1 to 18 to the skin.

23. Use of the composition according to any one of Claims 1 to 18 for preparing a salve or an ointment intended to therapeutically treat the human face and/or body.

24. Use of a crosslinked cationic polymer in order to solubilize an active agent in a composition containing an acidic hydrophilic medium, containing an amount of organic solvent representing from 20 to 90% of the total weight of the composition and including an amount of water representing not more than 45% of the total weight of the composition, and/or in order to gel the said composition.

## Patentansprüche

1. Zusammensetzung, die ein saures, hydrophiles Medium und mindestens einen Gelbildner, der aus einem vernetzten kationischen Polymer gebildet ist, enthält,
dadurch gekennzeichnet, daß
das hydrophile Medium ein Medium ist, das eine Menge an organischem Lösungsmittel enthält, die 20 bis 90 % des Gesamtgewichts der Zusammensetzung beträgt, und eine Wassermenge enthält, die höchstens 45 % des Gesamtgewichts der Zusammensetzung beträgt, wobei der Gelbildner der Zusammensetzung ein makroskopisch homogenes Aussehen eines Gels und Stabilität verleiht.

2. Zusammensetzung nach Anspruch 1, dadurch gekennzeichnet, daß der pH-Wert höchstens 4 beträgt.

3. Zusammensetzung nach Anspruch 1 oder 2, dadurch gekennzeichnet, daß die Wassermenge höchstens 30 % des Gesamtgewichts der Zusammensetzung beträgt.

4. Zusammensetzung nach einem der vorhergehenden Ansprüche, dadurch gekennzeichnet, daß das Polymer ein Homopolymer ist, daß durch Vernetzung mindestens eines ethylenisch ungesättigten kationischen Monomers oder mindestens eines seiner Salze mit einem mehrfach ethylenisch ungesättigten Vernetzungsmittel erzeugt wird.

5. Zusammensetzung nach einem der vorhergehenden Ansprüche, dadurch gekennzeichnet, daß das Polymer ein Polymer aus einem Dialkyldiaminoalkyl(meth)acrylat oder einem seiner Salze ist.

6. Zusammensetzung nach einem der vorhergehenden Ansprüche, dadurch gekennzeichnet, daß das Polymer ein Polymer aus einem gegebenenfalls quaternisierten Dimethylaminoethylmethacrylat ist.

7. Zusammensetzung nach einem der vorhergehenden Ansprüche, dadurch gekennzeichnet, daß das Polymer (bezogen auf die wirksame Substanz) in einem Anteil von 0,1 bis 10 % des Gesamtgewichts der Zusammensetzung enthalten ist.

8. Zusammensetzung nach einem der vorhergehenden Ansprüche, dadurch gekennzeichnet, daß das Polymer (bezogen auf die wirksame Substanz) in einem Anteil von 0,5 bis 6 % des Gesamtgewichts der Zusammensetzung enthalten ist.

9. Zusammensetzung nach einem der vorhergehenden Ansprüche, dadurch gekennzeichnet, daß das hydrophile Medium mindestens ein organisches Lösungsmittel enthält, das unter ein- oder mehrwertigen Alkoholen, gegebenenfalls ethoxylierten Polyethylenglykolen, Propylenglykolestern, Sorbitol und seinen Derivaten, Di-alkylisosorbiden, Glykolethern und Propylenglykolethern ausgewählt ist.

10. Zusammensetzung nach einem der vorhergehenden Ansprüche, dadurch gekennzeichnet, daß der Anteil des Lösungsmittels 20 bis 70 % des Gesamtgewichts der Zusammensetzung beträgt.

11. Zusammensetzung nach einem der vorhergehenden Ansprüche, dadurch gekennzeichnet, daß sie mindestens ein in dem hydrophilen Medium lösliches oder unlösliches Öl enthält.

12. Zusammensetzung nach einem der vorhergehenden Ansprüche, dadurch gekennzeichnet, daß der Anteil des Öls 0 bis 50 % des Gesamtgewichts der Zusammensetzung beträgt.

13. Zusammensetzung nach einem der vorhergehenden Ansprüche, dadurch gekennzeichnet, daß der Anteil des Öls 1 bis 15 % des Gesamtgewichts der Zusammensetzung beträgt.

14. Zusammensetzung nach einem der vorhergehenden Ansprüche, dadurch gekennzeichnet, daß sie außerdem mindestens einen depigmentierenden Wirkstoff enthält, der befähigt ist, die Haut des Menschen zu depigmentieren.

15. Zusammensetzung nach einem der vorhergehenden Ansprüche, dadurch gekennzeichnet, daß sie mindestens einen Wirkstoff enthält, der ausgewählt ist unter Ascorbinsäure, Kojisäure, Kaffeesäure, Salicylsäure und deren Derivaten, Glucuronsäure, Glykolsäure, Brenztraubensäure, 2-Hydroxybutansäure, 2-Hydroxypentansäure, 2-Hydroxyhexansäure, 2-Hydroxyheptansäure, 2-Hydroxyoctansäure, 2-Hydroxynonansäure, 2-Hydroxydecansäure, 2-Hydroxyundecansäure, 2-Hydroxydodecansäure, 2-Hydroxytetradecansäure, 2-Hydroxyhexadecansäure, 2-Hydroxyoctadecansäure, 2-Hydroxytetraecosansäure, 2-Hydroxyeicosansäure, Mandelsäure, Benzoesäure, Phenylmilchsäure, Gluconsäure, Galacturonsäure, Citronensäure, Elaeostearinsäure, Ribonsäure, Tartron-säure, Weinsäure, Äpfelsäure, Fumarsäure, Retinoesäure und deren Derivaten und Benzoldi-1,4- (3-methyliden-10-camphersulfonsäure).

16. Zusammensetzung nach einem der vorhergehenden Ansprüche, dadurch gekennzeichnet, daß sie mindestens einen Wirkstoff enthält, der unter Kojisäure, Kaffesäure, Salicylsäure und deren Derivaten sowie deren Gemischen ausgewählt ist.

17. Zusammensetzung nach einem der vorhergehenden Ansprüche, dadurch gekennzeichnet, daß sie außerdem mindestens einen Hilfsstoff enthält, der unter Ceramiden, Konservierungsmitteln, Antioxidantien, Emollientien und Parfüms ausgewählt ist.

18. Zusammensetzung nach einem der vorhergehenden Ansprüche, dadurch gekennzeichnet, daß sie aus einer kosmetischen und/oder dermatologischen Zusammensetzung besteht.

19. Kosmetische Verwendung der Zusammensetzung nach einem der Ansprüche 1 bis 18 für die Behandlung der Haut des Gesichts und/oder des menschlichen Körpers.

20. Kosmetische Verwendung der Zusammensetzung nach einem der Ansprüche 1 bis 18 für die Depigmentierung der Haut des Gesichts und/oder des menschlichen Körpers.

21. Verfahren zur kosmetischen Behandlung der Haut, dadurch gekennzeichnet, daß es darin besteht, auf die Haut eine Zusammensetzung nach einem der Ansprüche 1 bis 18 aufzutragen.

22. Kosmetisches Verfahren zur Depigmentierung der Haut des Gesichts und/oder des menschlichen Körpers, dadurch gekennzeichnet, daß es darin besteht, auf die Haut eine Zusammensetzung nach einem der Ansprüche 1 bis 18 aufzutragen.

23. Verwendung der Zusammensetzung nach einem der Ansprüche 1 bis 18 für die Herstellung einer Pomade oder einer Salbe, die für die therapeutische Behandlung des Gesichts und/oder des menschlichen Körpers bestimmt ist.

24. Verwendung eines vernetzten kationischen Polymers für die Solubilisierung eines Wirkstoffs in einer Zusammensetzung, die ein saures hydrophiles Medium enthält, das eine Menge eines organischen Lösungsmittels enthält, die 20 bis 90 % des Gesamtgewichts der Zusammensetzung beträgt, und das eine Wassermenge enthält, die höchstens 45 % des Gesamtgewichts der Zusammensetzung beträgt, und/oder für die Überführung der Zusammensetzung in ein Gel.
